# EUROPEAN PATENT SPECIFICATION

(11) **EP 3 738 576 B1**
(45) Date of publication and mention of the grant of the patent: **10.06.2026**
(21) Application number: 18899914.8
(22) Date of filing: 05.12.2018
(51) Int. Cl.: A61K 8/04, A61K 8/19, A61K 8/31, A61K 8/34, A61K 8/41, A61K 8/44, A61K 8/64, A61Q 1/00, A61Q 19/00

(54) **GEL-STATE COMPOSITION AND PRODUCTION METHOD THEREFOR**
GEL-ZUSAMMENSETZUNG UND VERFAHREN ZU IHRER HERSTELLUNG
COMPOSITION À L'ÉTAT DE GEL ET SON PROCÉDÉ DE PRODUCTION

(30) Priority: 12.01.2018 JP 2018003099
(43) Date of publication of application: 18.11.2020
(73) Proprietor: KANEKA CORPORATION, Osaka 530-8288 (JP)
(72) Inventor: TSUJI, Tadao, Settsu-shi, Osaka 566-0072 (JP)
(74) Representative: Hoffmann Eitle
(86) International application number: PCT/JP2018/044679
(87) International publication number: WO 2019/138739

(56) References cited:
- WO-A1-2005/020950
- WO-A1-2005/089708
- JP-A- 2000 087 006
- JP-A- 2003 212 750
- JP-A- 2005 162 741
- JP-A- 2006 265 153
- JP-A- S6 230 546
- US-A1- 2007 190 087

## Description

### TECHNICAL FIELD

The present invention relates to a gelatinous composition excellent in stability over time and a method for producing a gelatinous composition excellent in stability over time.

### BACKGROUND ART

Various dosage forms of cosmetics have been developed. The example of such dosage forms includes a gelatinous composition prepared by increasing the viscosity of an oleaginous component. A gelatinous composition is applied to various skin preparations and cosmetic preparations, such as cleansing cosmetics and hair cosmetics. A composition containing a polyvalent alcohol and a surfactant has been conventionally known as such a gelatinous composition prepared by increasing the viscosity of an oleaginous component. For example, a gelatinous composition containing surfactin as a biosurfactant, an analogous compound thereof or a salt thereof and a trivalent or more polyvalent alcohol is known (Patent document 1). A gelatinous composition is well compatible with skin and gives a smooth feeling during use, but has a problem with stability over time. For example, when a gelatinous composition is preserved in a gel state for a long time, an oleaginous component becomes separated.

In addition, an exothermic composition and calefacient cosmetics which may contain a biosurfactant is known (Patent document 2), and a skin preparation containing surfactin as a biosurfactant is known (Patent document 3).

Patent Document 4 describes an oil-in-water emulsified composition suitable for external preparation for skin and cosmetic product, which comprises 0.1 to 5% by mass of (A) lipopeptide compound derived from a microorganism such as surfactin and its analogous compound, 0.05 to 1.5 % by mass of (B) xanthan gum, 25 to 70 by mass of (C) oil component and (D) water, free from non-ionic surfactants and acrylic water-soluble polymers.

Patent Document 5 describes a method for easily producing an oily thickened gel-like composition which contains an anionic surfactant having a lipopeptide structure (a), water and/or a polyhydric alcohol (c), and an oily component (b). The method includes dispersing the anionic surfactant (a) in all or a part of the oily component (b). Further, the formed dispersion is mixed with the water and/or the polyhydric alcohol (c) in an amount of equal to or more than an amount of the dispersion, then the oily component (b), the water, and/or the polyhydric alcohol (c) are added to the formed mixture.

Patent Document 6 describes a cosmetic composition comprising 0.1 to 5 mass% of a lipopeptide compound, and 0.1 to 20 mass% of a polyoxyethylene glyceryl ether fatty acid ester and/or a polyoxyethylene sorbit fatty acid ester.

Patent Document 7 describes an oil-based thickening gel composition comprising (a) an anionic surfactant having a lipopeptide structure, (b) water and/or a polyhydric alcohol having a valence of 3 or more, (c) a tocopherol compound and (d) an oil component.

### PRIOR ART DOCUMENT

### PATENT DOCUMENT

Patent Document 1: JP 2003-176211 A
Patent Document 2: JP 2004-131413 A
Patent Document 3: JP 2004-67647 A
Patent Document 4: WO 2005/089708 A1
Patent Document 5: JP 2005/162741 A
Patent Document 6: WO 2005/020950 A1
Patent Document 7: US 2007/0190087 A1

### DISCLOSURE OF THE INVENTION

### PROBLEMS TO BE SOLVED BY THE INVENTION

The objective of the present invention is to provide a gelatinous composition excellent in stability over time, a skin preparation and a cosmetic preparation which contain the gelatinous composition, and a method for producing a gelatinous composition excellent in stability over time.

### MEANS FOR SOLVING THE PROBLEMS

The inventors of the present invention made extensive studies to solve the above problems. As a result, the inventors completed the present invention by finding that a gelatinous composition excellent in preservation stability can be obtained by adding the specific alkaline substance to a gelatinous composition containing a biosurfactant and a polyvalent alcohol.

The present invention is defined in the appended claims.

### EFFECT OF THE INVENTION

A gelatinous composition excellent in stability over time can be obtained according to the present invention.

### MODE FOR CARRYING OUT THE INVENTION

The gelatinous composition of the present invention contains a biosurfactant that is a salt of surfactin as discussed below. A biosurfactant is a natural compound which is produced by a microorganism. In general, a biosurfactant is characterized by extremely high safety to the environment and a human body, since a biosurfactant is highly biodegradable and has low skin irritation to a human body. The biosurfactant usable in the present invention is a lipopeptide compound biosurfactant since a gelatinous composition containing a cyclic lipopeptide biosurfactant tends to give a smooth feeling without rough feeling nor stickiness.

A salt of surfactin means the compound represented by the formula (1) . 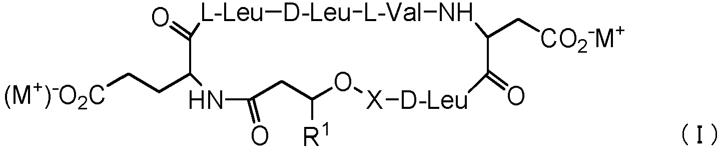 wherein
'X' is a residue of an amino acid selected from leucine, isoleucine and valine;
R¹ is a C₉₋₁₈ alkyl group;
'M⁺' is an alkali metal ion or a quaternary ammonium ion.

Although the amino acid residue as 'X' may be either in an L-form or a D-form, the L-form is preferred.

The term "C₉₋₁₈ alkyl group" means a linear or branched monovalent saturated hydrocarbon group having 9 or more and 18 or less carbon atoms. An example thereof includes n-nonyl, 6-methyloctyl, 7-methyloctyl, n-decyl, 8-methylnonyl, n-undecyl, 9-methyldecyl, n-dodecyl, 10-methylundecyl, n-tridecyl, 11-methyldodecyl, n-tetradecyl, n-pentadecyl, n-hexadecyl, n-heptadecyl and n-octadecyl.

The alkali metal ion is not particularly restricted, exemplified by a lithium ion, a sodium ion, a potassium ion or the like, and is preferably a sodium ion.

The example of a substituent of the quaternary ammonium ion includes an organic group, for example, an alkyl group such as methyl, ethyl, n-propyl, isopropyl, n-butyl and tert-butyl; an aralkyl group such as benzyl, methylbenzyl and phenylethyl; and an aryl group such as phenyl, toluyl and xylyl. An example of the quaternary ammonium ion includes a tetramethylammonium ion, a tetraethylammonium ion and a pyridinium ion.

One kind or two or more kinds of biosurfactants or salts thereof may be used.

The biosurfactant (i.e. salt of surfactin represented by the Formula (I) can be obtained by cultivating a microorganism which produces the target biosurfactant and separating the biosurfactant from a culture liquid of the microorganism in accordance with a conventional method. The purified biosurfactant may be used or the unpurified biosurfactant such as a culture liquid may be used. An example of a microorganism which produces surfactin includes a strain classified in Bacillus subtilis. The biosurfactant which is chemically synthesized can be also used similarly.

For example, a concentration of the biosurfactant in the gelatinous composition according to the present invention can be adjusted to 0.02 mass% or more and 3 mass% or less. When the concentration is 0.02 mass% or more, the stability over time of the gelatinous composition can be ensured more surely. When the concentration is 3 mass% or less, the deterioration of use feeling due to the excessive biosurfactant can be prevented more surely. The above concentration is preferably 0.05 mass% or more, more preferably 0.1 mass% or more, and preferably 2 mass% or less, more preferably 1.5 mass% or less.

The gelatinous composition of the present invention contains water and/or a polyvalent alcohol. The water and/or polyvalent alcohol means at least one selected from water and a polyvalent alcohol or one or more selected from water and a polyvalent alcohol, and the gelatinous composition of the present invention preferably contains both of water and a polyvalent alcohol. The polyvalent alcohol is not particularly restricted and preferably one or more selected from glycerin, sorbitol, xylitol, diglycerin and polyethyleneglycol to be used.

For example, a concentration of the water and/or polyvalent alcohol in the gelatinous composition of the present invention can be adjusted to 1 mass% or more and 50 mass% or less and preferably 1.5 mass% or more and 30 mass% or less. When the concentration of the water and/or polyvalent alcohol is included in the above range, the gelatinous composition with excellent use feeling can be produced.

When both of the water and polyvalent alcohol are used, a ratio thereof may be appropriately adjusted. For example, a ratio of the water to the total of the water and polyvalent alcohol can be adjusted to 40 mass% or more and 95 mass% or less, preferably 50 mass% or more and 90 mass% or less.

The gelatinous composition of the present invention contains an oleaginous component. The oleaginous component of the present invention is not particularly restricted as long as the oleaginous component is not mixed with water at an arbitrary ratio. More specifically, the gelatinous component means a substance which is not dissolved within 30 minutes when the substance is added to 1000 mL or more of water and the mixture is strongly shaken to be mixed at 20 ± 5°C for 30 seconds every 5 minutes. It is preferred to use one kind or two or more kinds selected from a hydrocarbon such as squalane, liquid paraffin, light liquid paraffin, ceresin, polyethylene powder, squalene, microcrystalline wax, vaseline, liquid isoparaffin, polybutene and mineral oil; wax such as beeswax, carnauba wax, candelilla wax, jojoba oil, lanolin and spermaceti; a fat and oil, such as macadamia nut oil, olive oil, cottonseed oil, soybean oil, avocado oil, rice bran oil, rice oil, rice germ oil, palm kernel oil, castor oil, rosehip oil, evening primrose oil, camellia oil, horse oil, grape seed oil, palm oil, meadowfoam seed oil, shea butter, corn oil, safflower oil and sesame oil; an ester such as ethylhexyl palmitate, isononyl isononanoate, isopropyl myristate, ethyl oleate, glyceryl tri(caprylate/caprate), cetyl 2-ethylhexanoate, glyceryl tri(2-ethylhexanoate), diisopropyl sebacate and cholesteryl hydroxystearate; a fatty acid such as myristic acid, stearic acid and oleic acid; a silicone oil such as methylpolysiloxane, methylphenylpolysiloxane and amino-modified silicone; a higher alcohol such as cetanol and oleyl alcohol; and an alkyl glyceryl ether such as batyl alcohol and chimyl alcohol.

A concentration of the oleaginous component in the gelatinous composition of the present invention may be adjusted to, for example, 50 mass% or more and 99 mass% or less. The concentration is preferably 51 mass% or more, and more preferably 70 mass% or more and 95 mass% or less. When the concentration of the oleaginous component is included in the above range, the gelatinous composition with excellent use feeling can be obtained.

The gelatinous composition of the present invention contains an alkaline substance. The alkaline substance is one or more kinds selected from a hydroxy group-containing amine compound, as defined below in §[46], an alkali metal hydroxide and a basic amino acid.

The hydroxy group-containing amine compound is one or more selected from triethanolamine, diethanolamine, monoethanolamine, diisopropanolamine, triisopropanolamine, 2-amino-2-methylpropanol, 2-amino-2-methylpropanediol, 2-amino-2-ethyl-1,3-propanediol, 2-amino-2-hydroxymethyl-1,3-propanediol or DL-1-amino-2-propanol, and more preferably triethanolamine, diethanolamine, monoethanolamine, diisopropanolamine, triisopropanolamine, 2-amino-2-methylpropanol, 2-amino-2-methylpropanediol, 2-amino-2-ethyl-1,3-propanediol or DL-1-amino-2-propanol.

The alkali metal hydroxide is preferably NaOH or KOH, and more preferably KOH.

The basic amino acid is preferably arginine, histidine or lysine, and more preferably lysine.

A concentration of the alkaline substance in the gelatinous composition of the present invention may be adjusted to, for example, 0.001 mass% or more and 2 mass% or less. The concentration is preferably 0.005 mass% or more, and preferably 1 mass% or less, more preferably 0.5 mass% or less. When the concentration of the alkaline substance is included in the above range, the gelatinous composition is stabilized.

Some amine compounds classified in the alkaline substance may form a salt with the biosurfactant which is an anionic surfactant; therefore, it is preferred to use a biosurfactant salt as the biosurfactant and to use the alkaline substance in addition to the biosurfactant salt for producing the gelatinous composition.

An optional component may be added to the gelatinous composition of the present invention as long as the effect of the present invention is achieved. An example of such an optional component includes a lower alcohol such as ethanol and isopropanol; an anionic surfactant, a cationic surfactant, an amphoteric surfactant, a thickener, an ultraviolet absorber, an antioxidant, an emollient agent, an emulsifier, a solubilizer, an anti-inflammatory drug, a humectant, a preservative, a disinfectant, a dye, a fragrance and a powder. It is preferred not to add a mineral such as zeolite to the gelatinous composition of the present invention.

When the optional component is used, a concentration of the optional component in the emulsion composition of the present invention may be adjusted depending on a kind of the optional component or the like and may be adjusted to, for example, 0.01 mass% or more and 10 mass% or less.

The pH of the gelatinous composition according to the present invention may be adjusted to, for example, 7.0 or more and 11.0 or less and is preferably 7.5 or more and 10.0 or less. The stability of the gelatinous composition can be expected to be further improved by adjusting the pH of the gelatinous composition to the above-described range.

A viscosity of the gelatinous composition according to the present invention may be appropriately adjusted and may be preferably adjusted to, for example, 10,000 mPa·s or more. The upper limit of the viscosity is not particularly restricted, and the viscosity may be adjusted to, for example, 100,000 mPa·s or less.

The gelatinous composition of the present invention can be produced by, for example, dissolving the biosurfactant and the alkaline substance in the water and/or polyvalent alcohol and adding the oleaginous component to the stirred mixture in small batches. When water and the polyvalent alcohol are used in combination, all or a part of the water may be added after the oleaginous component is added.

The oleaginous component may be added by a predetermined volume or in a continuous manner. Adding by a predetermined volume is referred to as divided addition, and adding in a continuous manner is referred to as continuous addition.
In a case of the divided addition, 60 mass% or less of the oleaginous component to the amount of the already added water and/or polyvalent alcohol is added at one time, and the mixture is stirred to be homogenous. The above ratio is preferably 30 mass% or less, and more preferably 10 mass% or less. The required amount of the oleaginous component is added by repeating this procedure. In a case of the continuous addition, an addition rate may be adjusted to 60 mass% or less of the amount of the already added water and/or polyvalent alcohol per minute. The addition rate is preferably 30 mass%/min or less and more preferably 10 mass%/min or less.

When the optional component is added, the optional component may be added by any methods; for example, the optional component may be added before the oleaginous component is added, dissolved or dispersed in the oleaginous component to be added, added after all of the oleaginous component is added, or added while the oleaginous component is added. All amount of the water and/or polyvalent alcohol may be added at first, or a part of the addition amount may be added and the remnant amount may be added later.

In particular, when water and the polyvalent alcohol are used in combination, it is preferred that the biosurfactant and the alkaline substance is added to all or a part of the water to obtain a first solution, the polyvalent alcohol is mixed with the first solution to obtain a second solution, and the oleaginous component or the remnant water and the oleaginous component are added to the second solution. The gelatinous composition produced by the steps has particularly excellent stability over time.

An example of a use application of the gelatinous composition according to the present invention preferably includes a skin preparation and a cosmetic preparation. For example, the gelatinous composition can be applied to a basic skin care such as a cream, a lotion, a cleansing gel and a cleansing cream; a cosmetic preparation for makeup, such as a foundation, an eye shadow, a lip color and a lip gloss; a hair care product such as a hair cream, a styling gel and hair wax; a cleaning product such as a shampoo, a hair conditioner, a hand cleanser, a body soap and a cleansing foam.

### EXAMPLES

For example, the gelatinous composition of the present invention is prepared as follows.

### Example 1

(A) surfactin sodium salt 0.67 mass%
(B) triethanolamine 0.0167 mass%
(C) glycerin 5 mass%
(D) squalane 91.67 mass%
(E) purified water 2.6433 mass%

A part of the purified water and the triethanolamine were added to the surfactin sodium salt, and the mixture was stirred to obtain 20 mass% surfactin aqueous solution. The glycerin was added to the surfactin aqueous solution, and the mixture was stirred to be mixed. Then the squalane and the remnant purified water were gradually added to the stirred mixture to obtain a gelatinous composition.

### Example 2

A gelatinous composition was produced similarly to Example 1 except that an amount of (B) component was 0.067 mass% and an amount of (E) component was 2.593 mass%.

### Example 3

A gelatinous composition was produced similarly to Example 1 except that 0.0033 mass% of diethanolamine was used as (B) component in place of triethanolamine and an amount of (E) component was 2.6567 mass%.

### Example 4

A gelatinous composition was produced similarly to Example 1 except that 0.01 mass% of diethanolamine was used as (B) component in place of triethanolamine and an amount of (E) component was 2.65 mass%.

### Example 5

A gelatinous composition was produced similarly to Example 1 except that 0.0033 mass% of monoethanolamine was used as (B) component in place of triethanolamine and an amount of (E) component was 2.6567 mass%.

### Example 6

A gelatinous composition was produced similarly to Example 1 except that 0.0083 mass% of diisopropanolamine was used as (B) component in place of triethanolamine and an amount of (E) component was 2.6517 mass%.

### Example 7

A gelatinous composition was produced similarly to Example 1 except that 0.0167 mass% of triisopropanolamine was used as (B) component in place of triethanolamine and an amount of (E) component was 2.6433 mass%.

### Example 8

A gelatinous composition was produced similarly to Example 1 except that 0.0067 mass% of 2-amino-2-methylpropanediol was used as (B) component in place of triethanolamine and an amount of (E) component was 2.6533 mass%.

### Example 9

A gelatinous composition was produced similarly to Example 1 except that 0.0067 mass% of 2-amino-2-ethyl-1,3-propanediol was used as (B) component in place of triethanolamine and an amount of (E) component was 2.6533 mass%.

### Example 10

A gelatinous composition was produced similarly to Example 1 except that 0.01 mass% of 2-amino-2-hydroxymethyl-1,3-propanediol was used as (B) component in place of triethanolamine and an amount of (E) component was 2.65 mass%.

### Example 11

A gelatinous composition was produced similarly to Example 1 except that 0.0067 mass% of DL-1-amino-2-propanol was used as (B) component in place of triethanolamine and an amount of (E) component was 2.6533 mass%.

### Example 12

A gelatinous composition was produced similarly to Example 1 except that 0.0033 mass% of KOH was used as (B) component in place of triethanolamine and an amount of (E) component was 2.6567 mass%.

### Example 13

A gelatinous composition was produced similarly to Example 1 except that 0.01 mass% of KOH was used as (B) component in place of triethanolamine and an amount of (E) component was 2.65 mass%.

### Example 14

A gelatinous composition was produced similarly to Example 1 except that 0.0267 mass% of lysine was used as (B) component in place of triethanolamine and an amount of (E) component was 2.6333 mass%.

### Example 15

A gelatinous composition was produced similarly to Example 1 except that 0.033 mass% of lysine was used as (B) component in place of triethanolamine and an amount of (E) component was 2.627 mass%.

### Example 16

A gelatinous composition was produced similarly to Example 1 except that 0.1167 mass% of histidine was used as (B) component in place of triethanolamine and an amount of (E) component was 2.5433 mass%.

### Comparative example 1

(A) surfactin sodium salt 0.67 mass%
(C) glycerin 5 mass%
(D) squalane 91.67 mass%
(E) purified water 2.66 mass%

A part of the purified water was added to the surfactin sodium salt, and the mixture was stirred to obtain 20 mass% surfactin aqueous solution. The glycerin was added to the surfactin aqueous solution, and the mixture was stirred to be mixed. Then the squalane and the remnant purified water were gradually added to the stirred mixture to obtain a gelatinous composition.

### Comparative example 2

A gelatinous composition was produced similarly to Example 1 except that 0.033 mass% of disodium hydrogenphosphate was used as (C) component in place of glycerin and an amount of (E) component was 2.627 mass%.

### Comparative example 3

A gelatinous composition was produced similarly to Example 1 except that 0.33 mass% of disodium hydrogenphosphate was used as (C) component in place of glycerin and an amount of (E) component was 2.33 mass%.

### Test example 1: Stability assessment

The gelatinous compositions of the above-described Examples 1 to 16 and Comparative examples 1 to 3 were statically placed at 50°C for up to 20 weeks, and then the appearances were evaluated on the basis of the following criteria. The results are shown in Table 1 and Table 2.

### Stability evaluation criteria

Good: gelatinous composition was maintained without separation.

Bad: gelatinous composition was separated as the oleaginous component was separated.

**Table 1**

| Component | | Ex.1 | Ex.2 | Ex.3 | Ex.4 | Ex.5 | Ex.6 | Ex.7 | Ex.8 | Ex.9 | Ex.10 | Ex.11 | Ex.12 | Ex.13 | Ex.14 | Ex.15 | Ex.16 |
|---|---|---|---|---|---|---|---|---|---|---|---|---|---|---|---|---|---|
| Surfactin Na | | 0.67 | 0.67 | 0.67 | 0.67 | 0.67 | 0.67 | 0.67 | 0.67 | 0.67 | 0.67 | 0.67 | 0.67 | 0.67 | 0.67 | 0.67 | 0.67 |
| Glycerin | | 5 | 5 | 5 | 5 | 5 | 5 | 5 | 5 | 5 | 5 | 5 | 5 | 5 | 5 | 5 | 5 |
| Squalane | | 91.67 | 91.67 | 91.67 | 91.67 | 91.67 | 91.67 | 91.67 | 91.67 | 91.67 | 91.67 | 91.67 | 91.67 | 91.67 | 91.67 | 91.67 | 91.67 |
| TEA | | 0.0167 | 0.067 | | | | | | | | | | | | | | |
| DEA | | | | 0.0033 | 0.01 | | | | | | | | | | | | |
| Monoethanolamine (2-Aminoethanol) | | | | | | 0.0033 | | | | | | | | | | | |
| Diisopropanolamine | | | | | | | 0.0083 | | | | | | | | | | |
| Triisopropanolamine | | | | | | | | 0.0167 | | | | | | | | | |
| 2-Amino-2-methylpropanediol | | | | | | | | | 0.0067 | | | | | | | | |
| 2-Amino-2-ethyl-1,3-propanediol | | | | | | | | | | 0.0067 | | | | | | | |
| 2-Amino-2-hydroxymethyl-1,3-propanediol | | | | | | | | | | | 0.01 | | | | | | |
| DL-1-Amino-2-propanol | | | | | | | | | | | | 0.0067 | | | | | |
| KOH | | | | | | | | | | | | | 0.0033 | 0.01 | | | |
| Lysine | | | | | | | | | | | | | | | 0.0267 | 0.033 | |
| Histidine | | | | | | | | | | | | | | | | | 0.1167 |
| Purified water | | 2.6433 | 2.593 | 2.6567 | 2.65 | 2.6567 | 2.6517 | 2.6433 | 2.6533 | 2.6533 | 2.65 | 2.6533 | 2.6567 | 2.65 | 2.6333 | 2.627 | 2.5433 |
| Total | | 100 | 100 | 100 | 100 | 100 | 100 | 100 | 100 | 100 | 100 | 100 | 100 | 100 | 100 | 100 | 100 |
| pH | | 8.033 | 8.506 | 7.535 | 7.96 | 7.985 | 7.804 | 7.813 | 8.114 | 7.664 | 7.863 | 7.82 | 7.717 | 10.177 | 8.581 | 9.778 | 7.662 |
| | 1W | Good | Good | Good | Good | Good | Good | Good | Good | Good | Good | Good | Good | Good | Good | Good | Good |
| | 4W | Good | Good | Good | Good | Good | Good | Good | Good | Good | Good | Good | Good | Good | Good | Good | Good |
| | 5W | Good | Good | Good | Good | Good | Good | Good | Good | Good | Good | Good | Good | \| Good | Good | Good | Good |
| | 6W | Good | Good | Good | Good | Good | Good | Good | Good | Good | Good | Good | Good | \| Good | Good | Good | Good |
| Stability assessment at 50°C | 8W | Bad | Good | Bad | Good | Good | Good | Good | Bad | Good | Good | Good | Good | Good | Good | Good | Bad |
| | 10W | | Bad | | Good | Good | Good | Good | | Good | Good | Good | Good | Good | Good | Good | |
| | 12W | | | | Good | Good | Good | Good | | Good | Bad | Good | Bad | Bad | Bad | Bad | |
| | 14W | | | | Bad | Bad | Good | Good | | Good | | Good | | | | | |
| | 18W | | | | | | Good | Bad | | Good | | Good | | | | | |
| | 20W | | | | | | Bad | | | Bad | | Bad | | | | | |

**Table 2**

| Component | | Comparative example 1 | Comparative example 2 | Comparative example 3 |
|---|---|---|---|---|
| Surfactin Na | | 0.67 | 0.67 | 0.67 |
| Glycerin | | 5 | 5 | 5 |
| Squalane | | 91.67 | 91.67 | 91.67 |
| TEA | | | | |
| DEA | | | | |
| Monoethanolamine (2-Aminoethanol) | | | | |
| Diisopropanolamine | | | | |
| Triisopropanolamine | | | | |
| 2-Amino-2-methylpropanediol | | | | |
| 2-Amino-2-ethyl-1,3-propanediol | | | | |
| 2-Amino-2-hydroxymethyl-1,3-propanediol | | | | |
| DL-1-Amino-2-propanol | | | | |
| KOH | | | | |
| Lysine | | | | |
| Disodium hydrogenphosphate | | | 0.033 | 0.33 |
| Purified water | | 2.66 | 2.627 | 2.33 |
| Total | | 100 | 100 | 100 |
| pH | | 7.33 | 7.522 | 8.018 |
| Stability assessment at 50°C | 1W | Good | Good | Good |
| | 4W | Bad | Bad | Bad |

As the results shown in Table 1 and Table 2, since the oleaginous component was separated in the gelatinous compositions of Comparative examples 1 to 3 after 4 weeks, the gelatinous compositions have a problem with preservation stability. On the one hand, the gelatinous compositions containing an alkaline substance of Examples 1 to 16 according to the present invention are excellent at preservation stability as the gelatinous compositions were maintained for more than 4 weeks.

## Claims

1. A gelatinous composition,
comprising a biosurfactant, an oleaginous component, an alkaline substance, and one or more selected from water and a polyvalent alcohol,
wherein the biosurfactant is a salt of surfactin represented by the formula (I): 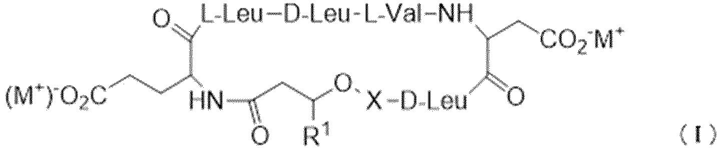
wherein
'X' is a residue of an amino acid selected from leucine, isoleucine and valine;
R¹ is a C₉₋₁₈ alkyl group;
'M⁺' is an alkali metal ion or a quaternary ammonium ion,
wherein the alkaline substance is one or more selected from a hydroxy group-containing amine compound, an alkali metal hydroxide and a basic amino acid,
wherein the hydroxy group-containing amine compound is one or more selected from triethanolamine, diethanolamine, monoethanolamine, diisopropanolamine, triisopropanolamine, 2-amino-2-methylpropanol, 2-amino-2-methylpropanediol, 2-amino-2-ethyl-1,3-propanediol, 2-amino-2-hydroxymethyl-1,3-propanediol and DL-1-amino-2-propanol, and
wherein a concentration of the oleaginous component is 50 mass% or more and 99 mass% or less.

2. The gelatinous composition according to claim 1, wherein the alkali metal hydroxide is one or more selected from NaOH and KOH.

3. The gelatinous composition according to claim 1 or 2, wherein the polyvalent alcohol is one or more selected from glycerin, sorbitol, xylitol, diglycerin and polyethyleneglycol.

4. The gelatinous composition according to any one of claims 1 to 3, wherein pH of the gelatinous composition is 7.0 or more and 11.0 or less.

5. A skin preparation, comprising the gelatinous composition according to any one of claims 1 to 4.

6. A cosmetic preparation, comprising the gelatinous composition according to any one of claims 1 to 4.

7. A method for producing a gelatinous composition,
wherein the gelatinous composition comprises a biosurfactant, water, an alkaline substance, a polyvalent alcohol, and an oleaginous component,
wherein the biosurfactant is a salt of surfactin represented by the formula (I): 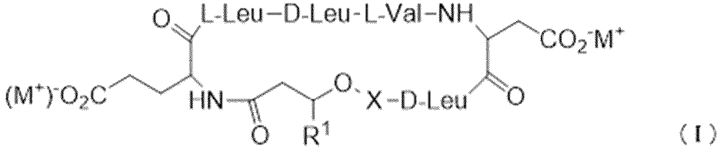 wherein
'X' is a residue of an amino acid selected from leucine, isoleucine and valine;
R¹ is a C₉₋₁₈ alkyl group;
'M⁺' is an alkali metal ion or a quaternary ammonium ion,
wherein the alkaline substance is one or more selected from a hydroxy group-containing amine compound, an alkali metal hydroxide and a basic amino acid,
wherein the hydroxy group-containing amine compound is one or more selected from triethanolamine, diethanolamine, monoethanolamine, diisopropanolamine, triisopropanolamine, 2-amino-2-methylpropanol, 2-amino-2-methylpropanediol, 2-amino-2-ethyl-1,3-propanediol, 2-amino-2-hydroxymethyl-1,3-propanediol and DL-1-amino-2-propanol, and
wherein a concentration of the oleaginous component is 50 mass% or more and 99 mass% or less,
comprising the steps of:
dissolving the biosurfactant and the alkaline substance in all or a part of the water to obtain a first solution,
mixing the first solution with the polyvalent alcohol to obtain a second solution, and
adding the oleaginous component or the remnant water and the oleaginous component to the second solution.

8. The method according to claim 7, wherein the alkali metal hydroxide is one or more selected from NaOH and KOH.

9. The method according to claims 7 or 8, wherein the polyvalent alcohol is one or more selected from glycerin, sorbitol, xylitol, diglycerin and polyethyleneglycol.

10. The method according to any one of claims 7 to 9, wherein pH of the gelatinous composition is 7.0 or more and 11.0 or less.

## Patentansprüche

1. Gallertartige Zusammensetzung,
umfassend ein Biotensid, eine ölhaltige Komponente, eine alkalische Substanz und eines oder mehrere, ausgewählt aus Wasser und einem mehrwertigen Alkohol,
worin das Biotensid ein Salz von Surfactin ist, dargestellt durch die Formel (I): 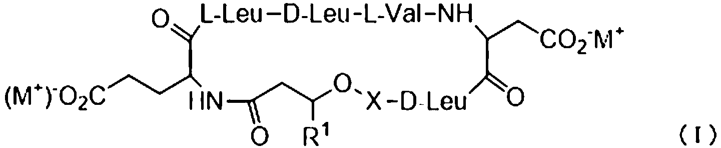
worin
'X' ein Rest einer Aminosäure ist, ausgewählt aus Leucin, Isoleucin und Valin;
R¹ eine C₉₋₁₈-Alkylgruppe ist;
'M⁺' ein Alkalimetallion oder in quartäres Ammoniumion ist,
worin die alkalische Substanz eine oder mehrere ist, ausgewählt aus einer Hydroxygruppen-haltigen Aminverbindung, einem Alkalimetallhydroxid und einer basischen Aminosäure,
worin die Hydroxygruppen-haltige Aminverbindung eine oder mehrere ist, ausgewählt aus Triethanolamin, Diethanolamin, Monoethanolamin, Diisopropanolamin, Triisopropanolamin, 2-Amino-2-methylpropanol, 2-Amino-2-methylpropandiol, 2-Amino-2-ethyl-1,3-propandiol, 2-Amino-2-hydroxymethyl-1,3-propandiol und DL-1-Amino-2-propanol, und
worin eine Konzentration der ölhaltigen Komponente 50 Massen-% oder mehr und 99 Massen-% oder weniger beträgt.

2. Gallertartige Zusammensetzung gemäß Anspruch 1, worin das Alkalimetallhydroxid eines oder mehrere ist, ausgewählt aus NaOH und KOH.

3. Gallertartige Zusammensetzung gemäß Anspruch 1 oder 2, worin der mehrwertige Alkohol einer oder mehrere ist, ausgewählt aus Glycerin, Sorbitol, Xylitol, Diglycerin und Polyethylenglycol.

4. Gallertartige Zusammensetzung gemäß einem der Ansprüche 1 bis 3, worin der pH der gallertartigen Zusammensetzung 7,0 oder mehr und 11,0 oder weniger ist.

5. Hautpräparat, umfassend die gallertartige Zusammensetzung gemäß einem der Ansprüche 1 bis 4.

6. Kosmetische Zusammensetzung, umfassend die gallertartige Zusammensetzung gemäß einem der Ansprüche 1 bis 4.

7. Verfahren zur Herstellung einer gallertartigen Zusammensetzung,
worin die gallertartige Zusammensetzung ein Biotensid, Wasser, eine alkalische Substanz, einen mehrwertigen Alkohol und eine ölhaltige Komponente umfasst,
worin das Biotensid ein Salz von Surfactin ist, dargestellt durch die Formel (I): 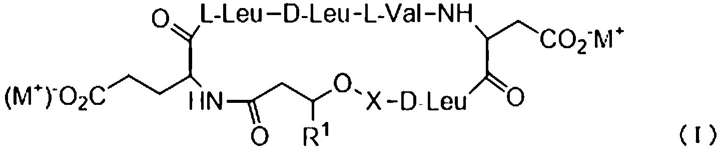
worin
'X' ein Rest einer Aminosäure ist, ausgewählt aus Leucin, Isoleucin und Valin;
R¹ eine C₉₋₁₈-Alkylgruppe ist;
'M⁺' ein Alkalimetallion oder in quartäres Ammoniumion ist,
worin die alkalische Substanz eine oder mehrere ist, ausgewählt aus einer Hydroxygruppen-haltigen Aminverbindung, einem Alkalimetallhydroxid und einer basischen Aminosäure,
worin die Hydroxygruppen-haltige Aminverbindung eine oder mehrere ist, ausgewählt aus Triethanolamin, Diethanolamin, Monoethanolamin, Diisopropanolamin, Triisopropanolamin, 2-Amino-2-methylpropanol, 2-Amino-2-methylpropandiol, 2-Amino-2-ethyl-1,3-propandiol, 2-Amino-2-hydroxymethyl-1,3-propandiol und DL-1-Amino-2-propanol, und
worin eine Konzentration der ölhaltigen Komponente 50 Massen-% oder mehr und 99 Massen-% oder weniger beträgt,
umfassend die folgenden Schritte:
das Lösen des Biotensids und der alkalischen Substanz in im gesamten Wasser oder einem Teil davon, so dass eine erste Lösung erhalten wird,
das Mischen der ersten Lösung mit dem mehrwertigen Alkohol, so dass eine zweite Lösung erhalten wird, und
das Zugeben der ölhaltigen Komponente oder des restlichen Wassers und der ölhaltigen Komponente zur zweiten Lösung.

8. Verfahren gemäß Anspruch 7, worin das Alkalimetallhydroxid eines oder mehrere ist, ausgewählt aus NaOH und KOH.

9. Verfahren gemäß Anspruch 7 oder 8, worin der mehrwertige Alkohol einer oder mehrere ist, ausgewählt aus Glycerin, Sorbitol, Xylitol, Diglycerin und Polyethylenglycol.

10. Verfahren gemäß einem der Ansprüche 7 bis 9, worin der pH der gallertartigen Zusammensetzung 7,0 oder mehr und 11,0 oder weniger ist.

## Revendications

1. Composition gélatineuse,
comprenant un biosurfactant, un composant oléagineux, une substance alcaline et un ou plusieurs éléments choisis parmi l'eau et un alcool polyvalent, dans laquelle le biosurfactant est un sel de la surfactine représenté par la formule (I) : 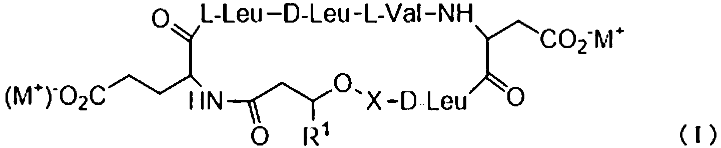
dans laquelle
« X » est un résidu d'un acide aminé choisi parmi la leucine, l'isoleucine et la valine ;
R¹ est un groupe alkyle en C₉₋₁₈ ;
« M⁺ » est un ion de métal alcalin ou un ion d'ammonium quaternaire,
dans laquelle la substance alcaline est un ou plusieurs composés choisis parmi un composé aminé contenant un groupe hydroxy, un hydroxyde de métal alcalin et un acide aminé basique,
dans laquelle le composé aminé contenant un groupe hydroxy est un ou plusieurs choisis parmi la triéthanolamine, la diéthanolamine, la monoéthanolamine, la diisopropanolamine, la triisopropanolamine, le 2-amino-2-méthylpropanol, le 2-amino-2-méthylpropanediol, le 2-amino-2-éthyl-1,3-propanediol, le 2-amino-2-hydroxyméthyl-1,3-propanediol et le DL-1-amino-2-propanol, et
dans laquelle la concentration du composant oléagineux est de 50 % en masse à 99 % en masse.

2. La composition gélatineuse selon la revendication 1, dans laquelle l'hydroxyde de métal alcalin est un ou plusieurs composés choisis parmi le NaOH et le KOH.

3. La composition gélatineuse selon la revendication 1 ou 2, dans laquelle l'alcool polyvalent est un ou plusieurs composés choisis parmi la glycérine, le sorbitol, le xylitol, le diglycérol et le polyéthylèneglycol.

4. La composition gélatineuse selon l'une quelconque des revendications 1 à 3, dans laquelle le pH de la composition gélatineuse est de 7,0 à 11,0.

5. Préparation pour la peau, comprenant la composition gélatineuse selon l'une quelconque des revendications 1 à 4.

6. Préparation cosmétique, comprenant la composition gélatineuse selon l'une quelconque des revendications 1 à 4.

7. Procédé de préparation d'une composition gélatineuse, dans lequel la composition gélatineuse comprend un biosurfactant, de l'eau, une substance alcaline, un alcool polyvalent et un composant oléagineux,
dans lequel le biosurfactant est un sel de surfactine représenté par la formule (I) : 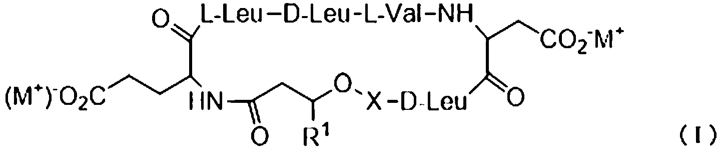
dans laquelle « X » est un résidu d'un acide aminé choisi parmi la leucine, l'isoleucine et la valine ;
R¹ est un groupe alkyle en C₉₋₁₈ ;
« M⁺ » est un ion de métal alcalin ou un ion d'ammonium quaternaire,
dans laquelle la substance alcaline est un ou plusieurs composés choisis parmi un composé aminé contenant un groupe hydroxy, un hydroxyde de métal alcalin et un acide aminé basique,
dans laquelle le composé aminé contenant un groupe hydroxy est un ou plusieurs choisis parmi la triéthanolamine, la diéthanolamine, la monoéthanolamine, la diisopropanolamine, la triisopropanolamine, le 2-amino-2-méthylpropanol, le 2-amino-2-méthylpropanediol, le 2-amino-2-éthyl-1,3-propanediol, le 2-amino-2-hydroxyméthyl-1,3-propanediol et le DL-1-amino-2-propanol, et
dans laquelle la concentration du composant oléagineux est de 50 % en masse à 99 % en masse,
comprenant les étapes suivantes :
dissolution du biosurfactant et de la substance alcaline dans tout ou partie de l'eau pour obtenir une première solution,
mélange de la première solution avec l'alcool polyvalent pour obtenir une deuxième solution, et
ajout du composant oléagineux ou l'eau résiduelle et le composant oléagineux à la deuxième solution.

8. Le procédé selon la revendication 7, dans lequel l'hydroxyde de métal alcalin est un ou plusieurs composés choisis parmi le NaOH et le KOH.

9. Le procédé selon les revendications 7 ou 8, dans lequel l'alcool polyvalent est un ou plusieurs composés choisis parmi la glycérine, le sorbitol, le xylitol, la diglycérine et le polyéthylèneglycol.

10. Le procédé selon l'une quelconque des revendications 7 à 9, dans lequel le pH de la composition gélatineuse est 7,0 ou plus et 11,0 ou moins.
